(19) Europäisches Patentamt
European Patent Office
Office européen des brevets

(11) Publication number: **0 139 891**
**A2**

(12) # EUROPEAN PATENT APPLICATION

(21) Application number: 84108867.7

(22) Date of filing: 26.07.84

(51) Int. Cl.$^4$: C 07 H 17/08
C 07 H 19/20, A 61 K 31/70

(30) Priority: 04.08.83 IT 2242783

(43) Date of publication of application:
08.05.85 Bulletin 85/19

(84) Designated Contracting States:
AT BE CH DE FR GB IT LI LU NL SE

(71) Applicant: B.T.B. INDUSTRIA CHIMICA S.p.A.
Via Paullo, 11
I-20067 Tribiano Milan(IT)

(71) Applicant: Fraschini, Franco
Via Caterina Da Forlì, 6
Milano(IT)

(72) Inventor: Anzani, Fulvio
Via Paullo, 11
Tribiano Milan(IT)

(72) Inventor: Viscardi, Renzo
Via Paullo, 11
Tribiano Milan(IT)

(72) Inventor: Pivoli, Franco
Via Paullo, 11
Tribiano Milan(IT)

(74) Representative: Bianchetti, Giuseppe
Studio Consulenza Brevettuale Via Senato 24
I-20121 Milan(IT)

(54) **Pharmaceutical compositions with antibiotic-immunostimulating activity.**

(57) Pharmaceutical compositions containing erythromycin, or an ester thereof, and inosine, or an acidic derivative thereof, in molar ratios varying from 5:1 to 1:5, particularly in the form of a salt of erythromycin dipropionate (or of erythromycin ethylsuccinate) with inosinic acid in the molar ratio 2:1, display antibiotic and immunostimulating properties, which make them particularly useful from the therapeutic point of view.

EP 0 139 891 A2

Croydon Printing Company Ltd.

# PHARMACEUTICAL COMPOSITIONS WITH ANTIBIOTIC-IMMUNOSTIMULATING ACTIVITY

The present invention refers to pharmaceutical compositions with antibiotic-immunostimulating activity, characterized in that they contain, as the active ingredient, erythromycin and inosine in molar ratios comprised between 5:1 and 1:5, preferably between 3:1 and 1:3. More particularly, the pharmaceutical compositions according to the invention contain, as the active ingredient, new salts between erythromycin and acidic derivatives of inosine in molar ratios 1:1 or 2:1, in which erythromycin may be present as such or in the form of the corresponding esters, as an example the propionate, the ethylsuccinate, the stearate and the analogs, and inosine may be present in the form of inosinic acid (hypoxanthine-riboside-5-phosphoric acid), inosine-5-diphosphoric acid, inosine-5-triphosphoric acid, or inosine-5-sulfuric acid (hypoxanthine-riboside-5-sulfuric acid).

A further object of the present invention is therefore represented by the salts of erythromycin (free or in the form of a corresponding ester) with acids deriving from inosine (5-phosphoric, 5-diphosphoric, 5-triphosphoric, 5-sulfuric) in molar ratios 1:1 or 2:1.

According to a preferred, though not limitative embodiment of the present invention, pharmaceutical compositions are claimed with antibiotic-immunostimulating properties, which contain, as the active

ingredient, the salt of erythromycin propionate with inosinic acid in the molar ratio 2:1. A particularly preferred, though not limitative aspect of the invention is thus represented also by the salt of etythromycin dipropionate and inosinic acid in the molar ratio 2:1, and by the salt of erythromycin ethylsuccinate and inosinic acid in the same molar ratio.

The pharmaceutical compositions according to the present invention proved surprisingly to be endowed with a concomitant antibacterial and immunostimulating activity. Recent studies on antibiotic therapies demonstrated that, when the antibiotic is present in the blood at high concentrations, the immunological defenses (phagocytosis, activity of NK cells) do not display any significant activity modification in comparison with that present in the same organism before the administration of the chemotherapeutic agent. When the antibiotic can no longer be dosed in the blood, the activity of the phagocytosis and the NK cells significantly increases and reaches very high values.

The pharmaceutical compositions of the present invention maintain the same antibacterial activity level and the same acute toxicity showed by erythromycin but, contrary to erythromycin, they immediately enhance the activity of the phagocytosis and the NK cells, also in the presence of the highest haematic concentrations of the antibiotic.

The activities of the phagocytosis and the NK

cells remain enhanced even after the decrease of the haematic concentrations of the antibiotic. Thus, the pharmaceutical compositions according to the invention may find useful therapeutic applications in all of those cases in which antibiotic treatments are required, thanks to the concomitant presence of the immunostimulating factors.

As stated above, preferred but not limitative compositions of the present invention are those containing, as the active ingredient, the salt of erythromycin propionate (or of erythromycin ethylsuccinate) with inosine-5-phosphoric acid in the molar ratio 2:1, hereinafter referred to, for brevity purposes, as F.F.001, respectively as F.F.002.

The following examples illustrate the preparation of the salts according to the invention.

EXAMPLE 1

79 Grams (0.1 mole) of erythromycin propionate and 17.4 g (0.05 mole) of inosinic acid were suspended in 300 ml of pure methanol and the resulting suspension was heated until complete dissolution. After evaporating the methanol *in vacuo*, a residue was obtained, which was taken up with 150 ml of anhydrous acetone; the suspension was filtered by suction and washed with 160 ml (2 x 80 ml) of anhydrous acetone, thus obtaining 91.5 g (yield: 95%) of a crystalline colourless compound of formula I:

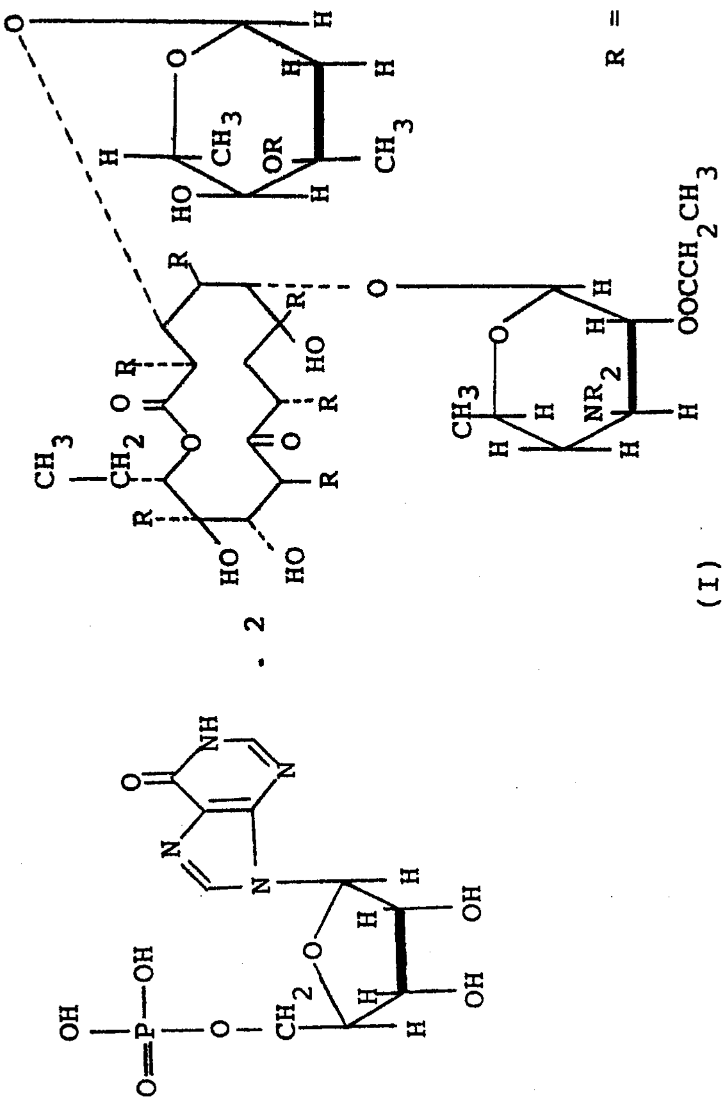

$R = CH_3$

(I)

which swells at 135°C and melts with decomposition at 168-174°C.

Elemental analysis: for $C_{90}H_{155}N_6O_{36}P$ (M.W.=1928.18)

| | %C | %H | %N | %O |
|---|---|---|---|---|
| calc. | 56.06 | 8.10 | 4.36 | 29.87 |
| trov. | 55.84 | 8.16 | 4.29 | 30.03 |

The spectroscopic and titration data confirm the proposed structure.

By operating as above described, and starting from equimolecular amounts of erythromycin propionate and inosinic acid, the corresponding 1:1 salt was prepared.

EXAMPLE 2

To the solution of 842 grams of erythromycin ethylsuccinate in 6000 ml of tetrahydrofuran, the solution of 170 grams of inosinic-5-phosphoric acid in 200 ml of water is added. The mixture is stirred for 15-20 minutes, then the solvent is evaporated *in vacuo* at 30-35°C. After complete drying, 1080 grams of the salt 2:1 are obtained, m.p. 140-148°C with swelling. $[\alpha]_D^{20} = -61.4°$.

By operating as above described, and starting

from the proper reactants in the suitable molar ratios, the following salts were obtained:

- erythromycin propionate/inosine-5-diphosphoric acid, molar ratio 2:1; erythromycin propionate/ inosine-5-diphosphoric acid, molar ratio 1:1; erythromycin propionate/inosine-5-triphosphoric acid, molar ratio 2:1; erythromycin propionate/ inosine-5-sulfuric acid, molar ratio 2:1; erythromycin stearate/inosinic acid, molar ratio 2:1; erythromycin stearate/inosine-5-diphosphoric acid, molar ratio 1:1; erythromycin ethylsuccinate/inosinic acid, molar ratio 1:1; erythromycin/ inosinic acid, molar ratio 1:1; erythromycin/ inosinic acid, molar ratio 2:1.

The following pharmaco-toxicological observations are referred to compound F.F.001, but in no way they must be considered as a limitation of the scopes of the present invention. Analogous qualitative data, though in some instances a little or quite different from the quantitative standpoint, were in fact obtained by testing other salts among those listed above, as well as mechanical mixtures of erythromycin, or esters thereof, and inosinic acid, or analogous derivatives of inosine.

Acute toxicity

The acute toxicity was determined on rats and mice after oral administration of F.F.001 in comparison with erythromycin base, administered at different dosages.

The $LD_{50}$ values in the rat are as follows:

| | |
|---|---|
| Erythromycin base | >6 g/kg |
| F.F.001 | >6 g/kg |

Antibacterial in vitro activity

The antibacterial *in vitro* activity of compound F.F.001, expressed as minimum inhibiting concentration (M.I.C.), was investigated versus the activity of erythromycin propionate. The M.I.C.s were determined against three pathogenic bacterial strains, and are expressed as micrograms of substance per ml of bacterial suspension ($\gamma$/ml).

| | F.F.001 | Er. prop. |
|---|---|---|
| S. pyogenes | 0.036 | 0.040 |
| S. aureus | 0.31 | 0.35 |
| H. influenzae | 0.31 | 0.31 |

Antibacterial in vivo activity

60 Sprague-Dawley rats of both sexes (3 lots of 20 animals each) were infected with an $LD_{100}$ of pneumococ bacteria. Said $LD_{100}$ was determined in $10^6$C.F.U. and was given to the rats through tracheal cannula. The death of the animals occurred at the 3rd-4th day. The observation period lasted 8 days. The compounds to be tested, i.e. F.F.001 and erythromycin propionate,as the comparison substance, were orally administered every twelve hours at a dosage of 25 mg/kg, expressed as erythromycin base.

A first group of 20 animals received salt F.F.001, to a second group the comparison substance was administered; a third group received nothing (control group). The obtained results are reported in the following Table I and are expressed as per cent mortali-

ties over the controls.

TABLE I

| Group | Treatment | Mortality |
|---|---|---|
| 1 | Control | 100% |
| 2 | Er. prop. | 5% |
| 3 | F.F.001 | 0% |

Both the dead and the survived animals underwent a hystological examination of the main organs, namely lungs, liver, kidneys, heart and brain.

Group 1 - controls - 100% deaths

The animals showed red hepatization of the lungs, intrahaepatic haemorrages and necrosis of the renal tubules.

Group 2 - Erythromycin - 5% deaths - 1 animal

The dead animal showed a telectasia of the right lung and tracheal perforation. The hystological examination of the other members of the group did not show anything abnormal.

Group 3 - F.F.001 - 0% deaths

Nothing to be pointed out.

In vivo activity in patients

Experimental schedule

The activity of salt F.F.001 was investigated in comparison with that of erythromycin propionate.

Therapeutical dosages

Erythromycin propionate - expressed as erythromycin base: 2 g/die.

F.F.001, 2.628 g corresponding to 2 g/die of erythromycin base, and to 0.473 g/die of inosine.

The antibacterial-immunostimulating activity of F.F.001 was investigated in a clinical research on patients affected by chronic bronchial infection. The immunostimulating action was determined through the monitorage of the phagocytary and NK cells activity.

Phagocytosis test

2 Ml of a phagocytes suspension ($10^6$ phagocytes/ml) was mixed up in a sterile test tube with 2 ml of St Albus ATCC 12228 ($10^6$ microorganisms/ml) suspended in Gelatine "Hanks-solution" with 10% AB serum and 0.4 ml of AB serum.

The mixture was then incubated at 37 degrees C under continuous stirring. 2 Ml of St Albus ATCC 12228 suspended in gelatine "Hanks-solution" with 10% of AB serum ($10^6$ microorganisms/ml) were mixed up in another sterile test tube with 2.04 ml of Gelatine "Hanks-solution" and incubated under the same conditions described above.

At regular time intervals (0-30 min.-60 min.-90 min.) aliquots of the suspension corresponding to 0.5 ml were added to 1.5 Ml of Gelatine "Hanks-solution" previously cooled to 4 degrees in order to stop the phagocytosis process.

The samples were then centrifuged for 4 min. at 110 r.p.m. in order to obtain the complete sedimentation of the leucocytes, while the upper phase consisted of a suspension containing the bacteria (supernatant).

The supernatant liquid was then diluted with a

sterile saline solution (Bacto-haemogglutination Soerensen buffer) at a 1:10 ratio, in order to be sure that at least the last dilution contained a number of living bacteria ranging between 100 and 1000/ml.

Aliquots of 0.1 ml of bacterial suspension were then taken from the three highest dilutions and both of them were pipetted on a plate containing Diagnostic Sensitive Test Agar (Oxoid) and immediately distributed on the whole surface with a sterile spatula.

The plates were incubated at 37 degrees C for 24 h and the number of colonies was then determined with a colony counter.

The number of living microorganisms per ml was then calculated from the mean value of the colonies which had developed on the plates (the test had been performed in duplicate), corresponding to the two highest dilutions, provided that the number of colonies did not exceed 500.

The same procedure was followed after having withdrawn aliquots of 0.5 ml of bacterial suspension from the test tube.

Calculations

Phagocytosis, as a function of time, was determined in vitro and can be expressed as the reduction percent of the number of living bacteria determined at the beginning of the experiment (No).

$$F(t) = \frac{No - Nt}{No} \times 100$$

F(t) is the phagocytosis index after t min., while Nt is the number of living bacteria after t min.

"Natural killer activity" test

This test consists in the determination of the percentage of aspecific lysis of a human tumoral target, K562, mediated by circulating lymphocytes.

The lymphocytes were isolated by centrifuging heparinized blood at a 1:1 dilution with physiologic solution or with a solution of "Hanks" salts of "Ficol".

After 30 min. of centrifugation at 1000 r.p.m. a band of stratified leucocytes was obtained over the "Ficol" level.

At the same time the K562 were labelled with $^{51}Cr$ by adding the radioisotope solution to a suspension containing $10^7$ cells in 0.5 ml of colture medium.

After 45 min. of incubation at 37 degrees C the cells were washed three times with a solution of "Hanks" salts.

The labelled cells were then incubated with leucocytes at 37 degrees C at various ratios for 4 h, after which the percentage of $^{52}Cr$ release was determined in the supernatant and assumed to be directly proportional to the K562 cell lysis mediated by leucocytes.

In Table II the results of phagocytosis test are reported.

Five groups each of 20 hospitalized patients,

affected of chronical bronchitis (age ranging from 40 to 54 years) were examined a) at the moment of administration respectively of 1) erythromycin; 2) inosine; 3) a mixture of erythromycin and inosine; 4) F.F.001; 5) F.F.002; and b) 2-4-6-8-24 hours after the administration.

In the groups 1), 3), 4) and 5) the dose of erythromycin was always 1 gram; in the groups 2), 3), 4) and 5) the corresponding dose of inosine was 182 mg.

TABLE II

| Hours | 0 | 2 | 4 | 6 | 8 | 24 |
|---|---|---|---|---|---|---|
| 1) Erythromycin | 8-24 | 8-24 | 8-24 | 8-24 | 31-60 | 8-24 |
| 2) Inosine | 5-19 | 10-50 | 5-19 | 5-19 | 5-19 | 5-19 |
| 3) Erythr. + Inos. | 4-18 | 12-60 | 8-24 | 8-24 | 30-61 | 4-18 |
| 4) F.F.001 | 5-20 | 16-62 | 16-62 | 16-62 | 34-82 | 5-20 |
| 5) F.F.002 | 5-20 | 16-62 | 16-62 | 16-62 | 34-82 | 5-20 |

The data of Table II show that phagocytosis is enhanced in case 1) after 8 hours; in case 2) after 2 hours; in case 3) after 2 and after 8 hours; in both cases 4) and 5), on the other hand, there is an increase at the 2nd hour; a "plateau" from the 2nd to the 6th hour; and a final peak at the 8th hour.

A similar behaviour is showed by the NK-test, whose results are reported in Table III relating to the same groups of patients and the doses speci-

fied in Table I.

TABLE III

| Hours | 0 | 2 | 4 | 6 | 8 | 24 |
|---|---|---|---|---|---|---|
| 1) Erythromycin | 7.4-23.0 | 7.4-23.0 | 7.4-23.0 | 7.4-23.0 | 45.3-67.8 | 7.4-23.0 |
| 2) Inosine | 4.5-19.0 | 10.0-56.5 | 4.5-19.0 | 4.5-19.0 | 4.5-19.0 | 4.5-19.0 |
| 3) Erythr. + Inos. | 5.2-19.4 | 10.8-55.4 | 10.8-36.0 | 10.8-36.0 | 44.0-68.2 | 5.2-19.4 |
| 4) F.F.001 | 4.9-21.7 | 45.8-68 | 47.3-70.1 | 48.0-71.1 | 50.2-74.2 | 4.9-21.7 |
| 5) F.F.002 | 5.2-17.8 | 44.9-68.2 | 46.1-69.8 | 50.1-71.8 | 52.9-76.1 | 5.2-17.8 |

The present invention also refers to all those industrially applicable acts and aspects connected with the employment of the above mentioned pharmaceutical composition in the quality of theerapeutic agents. An essential aspect of the present invention is thus represented by pharmaceutical formulations containing, as the active ingredient, predetermined amounts of erythromycin, and/or esters thereof, and inosine, and/or acidic derivatives thereof, in the form of the corresponding salts or mechanical mixtures. Said pharmaceutical formulations may contain from 100 to 1000 mg of erythromycin (as the free base) per dosage unit, as well as amounts of inosine, or acidic derivatives thereof, capable to satisfy the above reported molar ratios and, in particular, the preferred ones. Representative, but not limitative examples of these formulations are as follows:

- capsules for adults containing 660 mg of F.F.001, corresponding to 500 mg of erythromycin base;
- pediatric capsules containing 330 mg of F.F.001, corresponding to 250 mg of erythromycin base;
- bags containing 1370 mg of F.F.001, corresponding to 1 g of erythromycin base;
- granulated preparation containing 3% by weight of F.F.001 for extemporaneous suspension.

CLAIMS FOR BE,CH,DE,FR,GB,IT,LI,LU,NL,SE

1. Salts of erythromycin, or its ester, with acid derivatives of inosine.

2. Salts according to claim 1, in which the molar ratio erythromycin: acid derivative of inosine is comprised between 1:1 and 2:1.

3. Salt of erythromycine propionate with inosinic acid in a molar ratio 2:1.

4. Salt of erythromycine ethylsuccinate with inosinic acid in a molar ratio 2:1.

5. Pharmaceutical compositions with antibiotic-immunostimulating activity, containing a salt according to claims 1-4 as the active principle.

6. Pharmaceutical compositions with antibiotic-immunostimulating activity containing an association of a) erythromycine, or its esters, and b) inosine or an acid derivative thereof, as the active principle, the molar ratio between a) and b) being comprised between 5:1 and 1:5.

CLAIMS FOR AT

1. Process for the preparation of salts of erythromycin, or its esters, with acid derivatives of inosine, characterized by the fact that erythromycin, or an ester thereof, is reacted with an acid derivative of inosine in a solvent, and that the resulting salt is isolated by evaporation of the solvent.

2. Process according to claim 1, characterized by the fact that the molar ratio between erythromycin, or its ester, and the acid derivative of inosine is 2:1.

3. Process according to claim 1, characterized by the fact that the molar ratio between erythromycin, or its ester, and the acid derivative of inosine is 1:1.

4. Process according to claims 1 to 3, characterized by the fact that the erythromycin propionate is employed.

5. Process according to claims 1 to 3, characterized by the fact that the erythromycin ethylsuccinate is employed.

6. Process according to claims 1 to 5, characterized by the fact that the inosinic acid is employed.